# EUROPEAN PATENT SPECIFICATION

(11) **EP 1 035 780 B1**
(45) Date of publication and mention of the grant of the patent: **14.02.2007**
(21) Application number: 98961905.1
(22) Date of filing: 04.12.1998
(51) Int. Cl.: A23J 1/00, C12P 19/24, C12P 21/04, G01N 33/53

(54) **METHOD FOR PURIFYING HEAT SHOCK PEPTIDES COMPLEXES**
VERFAHREN ZUR REINIGUNG VON HITZE-SCHOCK PEPTIDKOMPLEXEN
PROCEDE SERVANT A PURIFIER DES COMPLEXES DE PEPTIDES ET DE PROTEINES DU STRESS

(30) Priority: 05.12.1997 US 985548; 05.12.1997 US 986234
(43) Date of publication of application: 20.09.2000
(73) Proprietor: The University of New Mexico, Albuquerque, NM 87131 (US)
(72) Inventor: WALLEN, Erik, S., Albuquerque, NM 87109 (US); MOSELEY, Pope, L., Albuquerque, NM 87122 (US)
(74) Representative: Klunker . Schmitt-Nilson . Hirsch
(86) International application number: PCT/US1998/025734
(87) International publication number: WO 1999/029182

(56) References cited:
- WO-A-98/12208
- S TAKEDA & D B MCKAY: "Kinetics of peptide binding to the bovine 70kDa heat shock cognate protein, a molecular chaperone" BIOCHEMISTRY, vol. 35, no. 14, 1996, pages 4636-4644, XP002130138 ISSN: 0006-2960
- PENG et al., "Purification of Immunogenic Heat Shock Protein 70-Peptide Complexes by ADP-Affinity Chromatography", JOURNAL OF IMMUNOLOGICAL METHODS, 1997, Vol. 204, pages 13-21, XP002917128
- GAO et al., "Effect of Constitutive 70kDa Heat Shock Protein Polymerization on Its Interaction with Protein Substrate", JOURNAL OF BIOLOGICAL CHEMISTRY, 12 July 1996, Vol. 278, No. 28, pages 16792-16797, XP002917129
- PALLEROS et al., "hsp70-Protein Complexes", JOURNAL OF BIOLOGICAL CHEMISTRY, 06 May 1994, Vol. 269, No. 18, pages 13107-13114, XP002917130
- SRIVASTAVA et al., "Heat Shock Protein-Peptide Complexes in Cancer Immunotherapy", CURRENT OPINIONS IN IMMUNOLOGY, 1994, Vol. 6, pages 728-732, XP002917131
- SRIVASTAVA P.K., "Peptide-Binding Heat Shock Proteins in the Endoplasmic Reticulum: Role in Immune Response to Cancer and in Antigen Presentation", ADVANCES IN CANCER RESEARCH, 1993, Vol. 62, pages 153-177, XP002917132
- SRIVASTAVA et al., "Heat Shock Proteins Transfer Peptides During Antigen Processing and CTL Priming", IMMUNOGENETICS, 1994, Vol. 39, pages 93-98, XP002917133

## Description

### RELATED APPLICATIONS

The present application is a continuation-in-part of U.S. Patent Application Nos. 08/717,239 (U.S. Patent No. 5,747,332), and 08/934,139, the entire disclosure and contents of which are hereby incorporated by reference.

### BACKGROUND OF THE INVENTION

### Field of the Invention

The present invention relates generally to methods for synthesizing heat shock protein-peptide complexes. The present invention also relates generally to methods for treating allergic diseases such as asthma and skin rashes using heat shock protein-based complexes.

### Description of the Prior Art

Heat shock proteins (HSPs) are associated in cells with a broad spectrum of peptides, polypeptides, denatured proteins and antigens with which they form complexes. These complexes form as part of the cell's normal protein manufacturing process as described in Beckmann *et al*., "Interaction of Hsp70 with newly synthesized proteins: implications for protein folding and assembly" in *Science* (1990), 248:850-4. HSP-peptide complexes may also be formed as part of the transport of peptides to the MHC class I molecules on the cell's surface as described in Srivastava *et al*., "Heat shock proteins transfer peptides during antigen processing" in *Immunogenetics* (1994), 39:93-98. When purified from autologous tumors, such HSP-peptide complexes have been useful in vaccines against cancers and infectious diseases as described in Srivastava *et al*., "Heat shock protein-peptide complexes in cancer immunotherapy" in *Current Opinion in Immunology* (1994), 6:728-732; and Srivastava, "Peptide-Binding Heat Shock Proteins in the Endoplasmic Reticulum" in *Advances in Cancer Research* (1993), 62:153-177. Vaccination with antigenic peptides alone can elicit an immune response but peptides bound to HSPs appear to elicit a much more efficient response. In this regard, Srivastava has estimated in Srivastava, "Heat shock proteins in immune responses to cancer: the fourth paradigm" in *Experientia* (1994), 50:1054-1060, that 10µg of gp96 isolated from tumor cells may be complexed with approximately 10pg of peptide representing a tumor-associated mutation. While 10pg of peptide cannot be used to successfully vaccinate an animal against a subsequent tumor challenge, 10pg of peptide complexed with gp96 is enough to elicit protection.

Several types of cancer vaccines that are currently being investigated, including heat shock protein vaccines, have been described in Old, "Immunotherapy for cancer" in *Scientific American* (1996), 275:142. These potential vaccines have included: inactivated cancer cells and their extracts which can jump-start the immune system (particularly cancer cells engineered to secrete cytokines, such as IL-2 or GM-CSF, which similarly heighten anti-tumor immunity, and cells designed to express co-stimulatory molecules, such as B-1, which enhance the ability of T-cells to recognize tumor cells); tumor peptides, fragments of tumor proteins recognized by T-cells, which can be injected alone or with immune-boosting adjuvants; injected tumor proteins which are taken up by antigen-presenting cells and are broken down into a range of peptide fragments recognized by T-cells; dendritic cells, antigen-presenting cells which are isolated from the blood, exposed to tumor peptides or engineered to produce tumor proteins and then reinjected; gangliosides, which humans can produce antibodies against and which are found on the surface of tumor cells; viral and bacterial vectors, genes coding for tumor antigens which are incorporated into viral or bacterial genomes, that, when injected, draw immunity against themselves and encoded antigens; and DNA and RNA coding for tumor antigens which prompt normal cells to begin producing these antigens.

The heat shock protein vaccines consist of HSP-peptide complexes purified from tumor cells containing a mixture of peptides as described by Udono and Srivastava in "Heat shock protein 70-associated peptides elicit specific cancer immunity" in *Journal of Experimental Medicine* (1993), 178:1391-6. Most of the peptides complexed with HSPs and isolated from tumor cells are presumably portions of normal cellular proteins. Therefore, the HSP-peptide complexes need to be purified at fairly high levels in order to obtain HSPs complexed with peptide portions of mutated oncogenes. Because many of the purified peptides are portions of normal cellular proteins, vaccination with these purified complexes may incur the risk of auto-immune disorders.

Peptide vaccination experiments use peptide sequences of known tumor mutations such as described in Gjertsen *et al*., "Vaccination with mutant ras peptides and induction of T-cell responsiveness in pancreatic carcinoma patients carrying the corresponding RAS mutation" in *Lancet* (1995), 346:1399-1400. Peptide vaccination experiments are also being tried with viral diseases such as described in Kelleher *et al*., "Safety and immunogenicity of UB1 HIV-IMN octameric V3 peptide vaccine administered by subcutaneous injection" in *AIDS Research & Human Retroviruses* (1997), 13:29-32 are currently receiving much attention.

HSP-peptide complexes have been purified from autologous tumors and used for vaccination against the same tumor. It has been anticipated that material purified in this way can also be used for vaccination against human tumors in Srivastava and Udono, "Heat shock protein-peptide complexes in cancer immunotherapy" in *Current Opinion in Immunology* (1994), 6:728-32. The use of these purified complexes in treating patient tumors would require the isolation of tumor material from each individual patient. This is not practical or even possible for many types of tumors. Many types of cancer, however, have well-defined mutations in specific genes. The use of *de novo* HSP-peptide complexes allows the use of HSP-peptide vaccines without the need for removing a portion of the tumor. The *de novo* complexes also allow prophylactic vaccination against a variety of diseases using peptides representing known oncogenic mutations or peptides representing portions of known viral proteins.

Bacterial hsp70-peptide complexes, created by incubating purified HSPs together with a synthetic peptide solution have also been used to stimulate peptide specific T-cell responses (See Roman *et al*., "Synthetic peptides non-covalently bound to bacterial hsp70 elicit peptide specific T-cell responses *in vivo," Immunology,* (1996), 88:487-492, and Roman *et al*., "Delayed-type hypersensitivity elicited by synthetic peptides complexed with *mycobacterial tuberculosis* hsp70," *Immunology,* (1997), 90:52-56).

In general, previous heat shock protein purifications have had two goals. The first goal has been to obtain pure HSPs without any contaminating peptides or proteins as described in Welch *et al*., "Rapid purification of mammalian 70,000-dalton stress proteins: affinity of the proteins for nucleotides" in *Molecular and Cellular Biology* (1985), 5:1229-1237 and in Nandan *et al*., "A rapid single-step purification method for immunogenic members of the hsp family: validation and application" in *Journal of Immunological Methods* (1994), 176:255-263. The second goal has been to purify the intracellular HSP-peptide complexes as described in Udono *et al*., "Heat shock protein 70-associated peptides elicit specific cancer immunity" in *Journal of Experimental Medicine* (1993), 178:1391-1396.

However, the fact that previously described methods only produce HSPs already complexed with intracellular peptides or stripped of their associated peptides is a serious deficiency. Also, there is no known simple way of synthesizing purified HSP-peptide complexes.

Numerous ways have been used in the past to treat allergic diseases such as desensitization therapy using allergy shots. For example, in the case of an individual suffering allergic rhinitis, the patient is given allergy shots containing antigenic peptides or proteins which appear to shift the response from IgE to IgG production or to induce T-Cell mediated suppression (See Kuby, J., *Immunology,* (New York: W.H. Freeman, 1994) p. 432 However, the number of allergic diseases which can be treated using desensitization therapy is limited and it has been shown to have no value in treating many allergic diseases such as asthma.

The current mainstay of allergic disease therapy is the administration of topical (including inhaled) or system corticosteroids. These drugs work by globally blunting the immune response in an individual. Unfortunately, they have severe side effects. Because systemic corticosteroids blunt the entire immune response, they immunosuppress a patient increasing their vulnerability to infection. Corticosteroids also cause cataracts, bone demineralization, muscle wasting, weight gain, fluid and sodium retention, and mood disturbances such as insomnia, and steroid psychosis. Even inhaled steroids have been shown to retard growth in children and to cause a loss of calcium from the bones.

Other treatments for allergic diseases include the use of antihistamines and cromalyn like agents. However, these treatments only treat the symptoms caused by an allergic disease and do not address the immune response itself.

Therefore, there exists a need for an effective treatment of allergic diseases which can be used to treat numerous diseases and which does not have undesirable side effects.

### SUMMARY OF THE INVENTION

The present invention provides a method of creating HSP-peptide combinations that will increase the effectiveness of peptide-based vaccines and allow more flexibility of use than the current HSP-based vaccines which require isolation of material from a portion of the tumor itself. Although several authors have described systems in which HSP vaccines might be used, including: Blachere *et al*., "Heat shock protein vaccines against cancer" in *Journal of Immunology* (1993), 14:352-56; Heike *et al*., "Heat shock protein-peptide complexes for use in vaccines" in *Journal of Leukocyte Biology* (1996), 60:153-8; and Srivastava, "Heat shock proteins in immune response to cancer: the fourth paradigm" in *Experientia* (1994), 50:1054-60; Roman *et al*., "Synthetic peptides non-covalently bound to bacterial hsp70 elicit peptide specific T-cell responses *in vivo," Immunology,* (1996), 88:487-492; Roman *et al*., "Delayed-type hypersensitivity elicited by synthetic peptides complexed with *mycobacterial tuberculosis* hsp70, *Immunology,* (1997), 90:52-56; none of these authors have speculated on the usefulness of purifying the proteins and synthesizing the complexes in a single step.

In one embodiment, the present invention provides a method for synthesizing heat shock protein complexes comprising the steps of: adding a heat shock protein to a denatured protein matrix to bind the heat shock protein to the denatured protein matrix and adding a complexing solution comprising a peptide to elute a heat shock protein-peptide complex.

The present invention further provides a heat shock protein-peptide complex made according to the method of the invention. Preferably, the heat shock protein-peptide complex is an ADP-heat shock protein-peptide complex.

The present invention further provides an apparatus for synthesizing ADP-heat shock protein-peptide complexes comprising an ADP-heat shock protein complex bound to a denatured protein matrix.

It is an object of the present invention to provide a treatment for allergic diseases which channels the immunoresponse rather than blunting it to leave a patient with a functional immune system to fight infections.

It is another object of the present invention to provide a treatment for allergic diseases which does not have severe side effects.

It is yet another object of the present invention to provide a treatment for allergic diseases which treats the allergic immune response directly, rather than just the symptoms.

The present invention provides a method for treating an allergic disease in which a heat shock protein-antigen complex is administered to a mammal in an amount sufficient to reduce the susceptibility of the mammal to a Th2 response for the allergic disease. The method of the present invention can be used either to prevent an individual from having an allergic reaction to an allergic disease or to reduce the effects of an allergic disease in an individual already suffering from the allergic disease.

Other objects and features of the present invention will be apparent from the following detailed description of the preferred embodiments.

### BRIEF DESCRIPTION OF THE DRAWINGS

The invention will be described in conjunction with the accompanying drawings, in which:

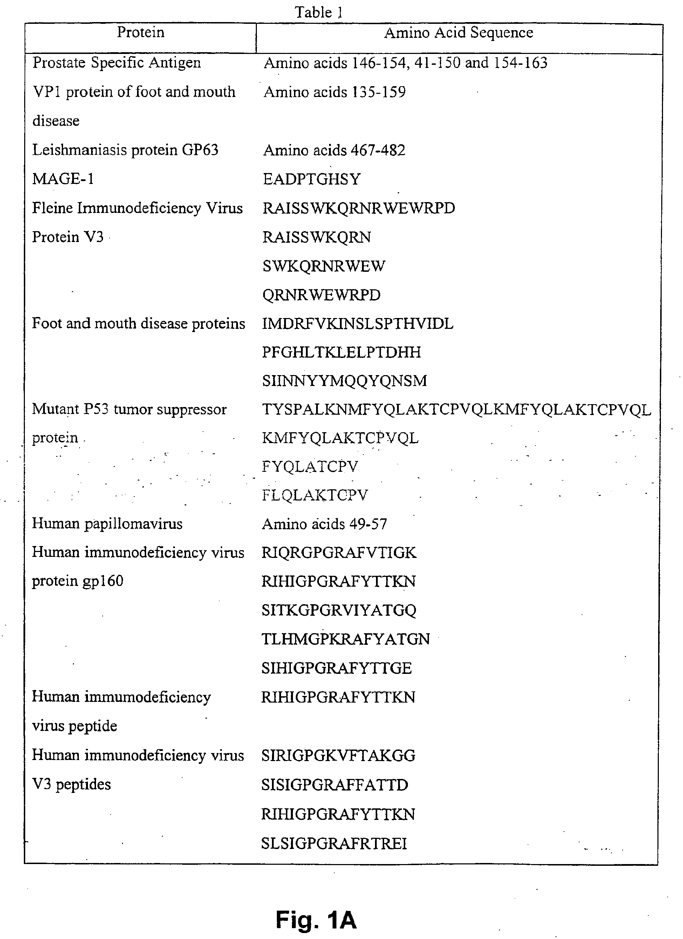

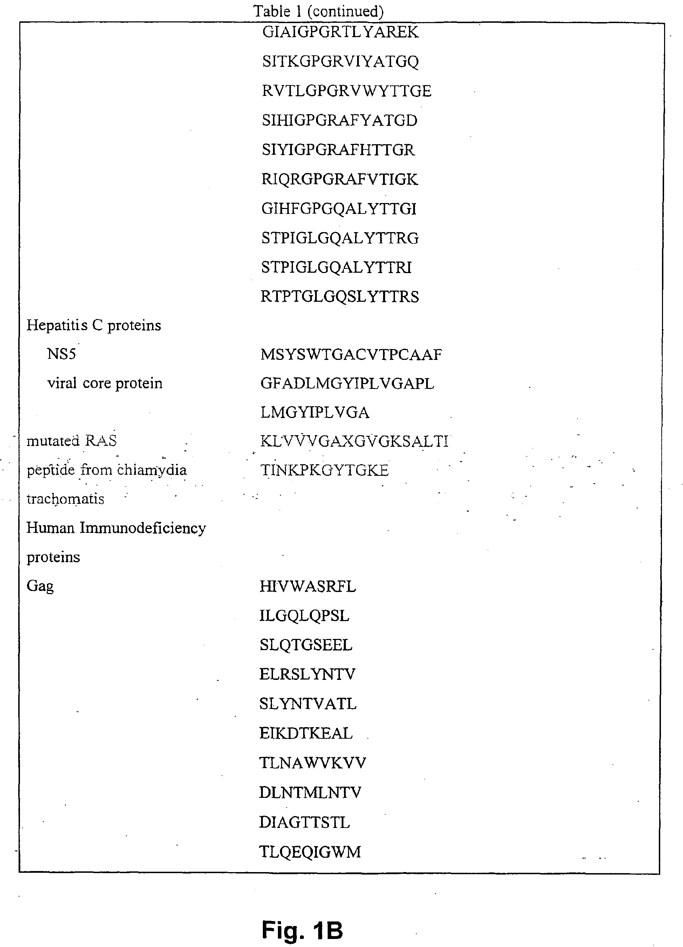

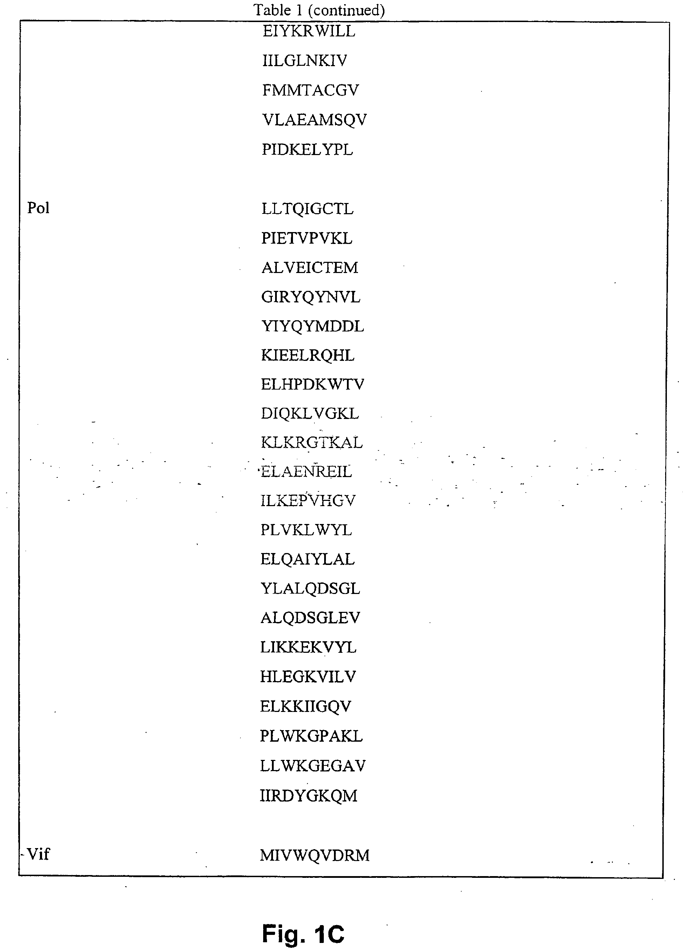

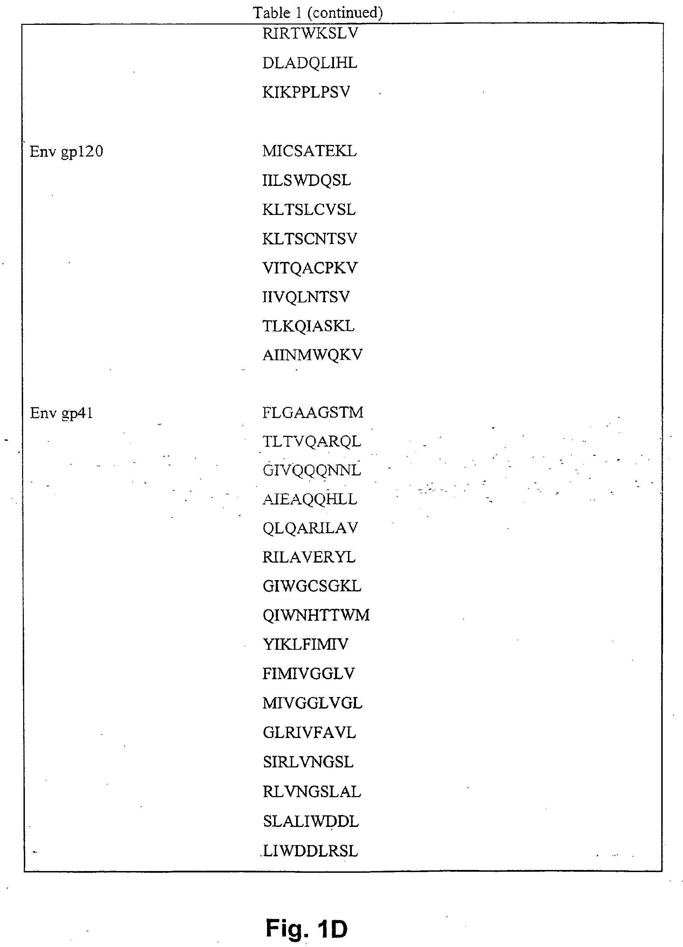

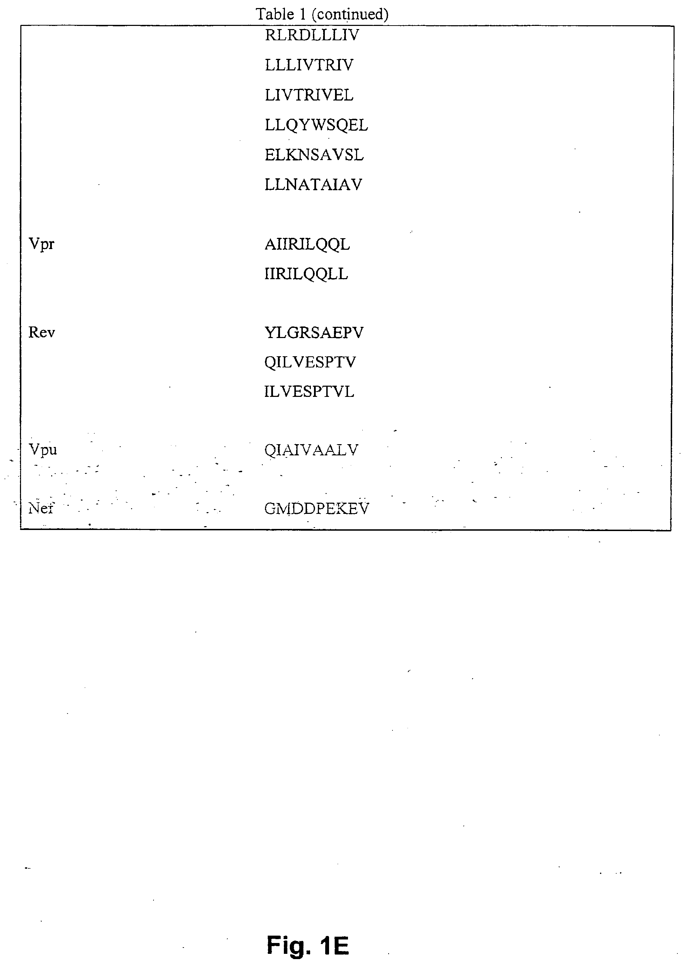

Figures 1A to 1E are a table showing peptides suitable for use in a preferred embodiment of the invention.

### DETAILED DESCRIPTION OF THE PREFERRED EMBODIMENTS

### Definitions

For the purposes of the present invention the term "peptide" refers to all types of peptides and conjugated peptides including: peptides, proteins, polypeptides, protein sequences, amino acid sequences, denatured proteins, antigens, oncogenes and portions of oncogenes.

For the purposes of the present invention the term "individual" refers to either an individual person or animal from whom a cell lysate, heat shock protein, or peptide is obtained or an individual patient who is treated according to the method of the present invention.

For the purposes of the present invention the term "allergic disease" is used broadly to cover reactions to various antigens which cause a Th1 response and includes such diseases as asthma, allergic rhinitis, and sinsusitis; allergic conditions of the lung such as eosinophilic pneumonia; allergic diseases of the skin, such as skin rashes due to reactions to poison ivy, poison oak, bee stings, jellyfish stings, etc.; and allergic diseases of other organs such as the eye, kidney, intestine, liver, etc.

For the purposes of the present invention, the term "mammal" includes human beings.

For the purposes of the present invention the term "antigen" includes peptide and nonpeptide substances capable of inducing an immune response.

### Description

In a preferred embodiment the invention provides a method for synthesizing HSP-peptide complexes. In this method, HSP complexes are bound to a denatured protein matrix or column. Then a complexing agent, such as a peptide, a polypeptide, a denatured protein and/or an antigen is added to the denatured protein column to bind with the HSP complexes, thereby removing these complexes from the column and forming an HSP-peptide complex which is eluted.

A preferred denatured protein matrix for the present invention can be formed by adding gelatin-agarose beads to a standard chromatography column.

One method for obtaining purified heat shock proteins for the present invention is to use a gelatin-agarose method such as that described in *Nandan et al*., "A rapid single-step purification method for immunogenic members of the hsp family; validation and application," in *Journal of Immunological Methods* (1994), 176:255-263. In this method of purification, cell lysates containing heat shock proteins are mixed with gelatin-agarose beads to bind the heat shock proteins to the beads. ATP is then added to the beads to release purified heat shock proteins.

Instead of synthesizing pure HSP-peptide complexes, it is often desirable to elute the HSP-peptide complex as an ADP-HSP-peptide complex. As described in U.S. Patent No. 5,747,332, such ADP-HSP-peptide complexes are stable and may be used to develop vaccines or immunotherapeutic tools for tumors and for infectious diseases. These ADP-HSP-peptide complexes can be formed in a number of ways. Preferably such complexes are formed by adding the heat shock protein to the denatured protein matrix in the form of an ADP-heat shock protein complex so that the HSP-peptide complexes synthesized in the column will include complexed ADP. To form the ADP-heat shock protein complex, the purified heat shock protein is mixed with ADP. A solution containing the ADP-heat shock protein complex is then added to an agarose-gelatin matrix to bind the heat shock protein to the denatured protein or gelatin. Because the denatured protein in the column binds to a different site on the heat shock protein than the ADP does, the ADP remains complexed to the heat shock protein, even when it is bound to the denatured protein in the column. A peptide complexing solution containing a peptide complexing agent is then added to the column to release the ADP-HSP complex as an ADP-HSP-peptide complex by binding to the peptide binding site on the HSP.

In addition to the-process described above, there are a number of other ways of insuring that ADP is complexed to at least some of the HSP-peptide complexes that are eluted. For example, ADP can be added to an HSP-containing solution, such as a cell lysate, before, after or at the same time as the HSP-containing solution is added to the denatured protein matrix. Also, where the HSP is added to the matrix as part of a solution or cell lysate containing ATP, ADP-HSP-peptide complexes can be synthesized by converting ATP to ADP in the cell lysate either before or after it is added to the matrix. One method for converting ATP to ADP in a cell lysate is to add glucose and hexokinase to the lysate containing ATP and let the lysate sit a room temperature for a period of time. Other suitable methods for converting ATP to ADP are described in Lehninger, *Biochemistry,* (New York: Worth Publishers, 1970), p. 299.

Some of the peptides suitable for use as peptide complexing agents in the present invention include those described in U.S. Patent Nos. 5,348,864 (vav mouse oncogene); 5,320,941 (protein sequence of the mas oncogene and polypeptides derived therefrom); 5,614,192 (peptides capable of binding to T-cell receptors); and 5,550,214 (peptide capable of binding in the HLA-A2 binding cleft and capable of stimulating proliferation of cytotoxic T-lymphocytes); the entire disclosure and contents of which are hereby incorporated by reference. Other suitable peptides are listed in Table 1 of Figures 1A to 1E.

The peptide complexing agents are typically obtained from purified cell lysates. The cells from which the peptide complexing agents are obtained can be from the same or different individual as the heat shock proteins to which they are complexed. The heat shock protein and peptide complexing agents can even be obtained from different species and either the heat shock protein or peptide complexing agent can be formed synthetically.

Although there are many heat shock proteins that may be used in the method of the present invention, heat shock proteins that have proven particularly useful include members of the hsp60 family, hsp70 family, hsp90 family and the hsp104-105 family.

Members of the hsp60 family include hsp60, hsp65, rubisco binding protein, and TCP-1 in eukaryotes; GroEl/GroES in prokaryotes; and Mif4 and TCP1alpha and beta in yeast.

Members of the hsp70 family include DnaK proteins from prokaryotes, Ssa, Ssb, and Ssc from yeast, hsp70, Grp75 and Grp78(Bip) from eukaryotes.

Members of the hsp90 family include hsp90, g96 and grp94.

Members of the hsp104-105 family include hsp105 and hsp110.

In another preferred embodiment, the present invention provides an apparatus for synthesizing heat shock protein-peptide complexes. Such an apparatus may be created by adding a desired heat shock protein or ADP-heat shock protein complex to a denatured protein matrix, as described above for the method of the present invention, to form a column having heat shock proteins bound therein. Such a column can be used to form a desired HSP-peptide complex or ADP-HSP-peptide complex by adding a peptide complexing agent to the column to elute the HSP-peptide complex or ADP-HSP-peptide complex, respectively, as described above for the method of the present invention.

In addition, the present invention provides a method for treating asthma and allergic diseases using heat shock proteins (HSPs) and HSP-antigen complexes. The method is particularly useful where the antigen is a peptide.

The treatment is based on the fact that there are two categories of response to an antigen. One is a cell mediated response (Th1) and the other is a T-helper cell activation of lymphocytes resulting in an allergic type antibody mediated response (Th2). The response to any particular antigen tends to be either a Th1 or a Th2 response since each response down regulates the other.

The response of the immune system (Th1 or Th2) to an antigen is based in part on how the antigen is presented to the immune system. Soluble antigen, taken up by antigen presenting cells (APCs) and displayed on the surface of APCs results in a Th2 response to that antigen. Cell mediated responses are more complex and require the recognition of intracellular antigens displayed on the surface of the target cell.

It has been speculated that an organism's own HSPs serve as a danger signals which can alert the cell to the presence of viral infections or transformed cells (See Matzinger *et al*., "Tolerance, danger, and the extended family, *Annual Review of Immunology,* (1994), v.12, pp. 991-1045). It is believed that these danger signals, especially when they come from within the same species (i.e. human HSPS in a human, mouse in a mouse, etc.), favor a cell mediated response to an antigen (See Srivastava *et al*., "Immunotherapy of tumors with autologous tumor-derived heat shock protein preparations," *Science,* (1997), 278, 117-120). Therefore, allergy causing antigens presented as complexes with HSPs should result in a cellular reaction to those antigens which favors a Th1 environment and down regulates any Th2 response to that antigen.

The heat shock protein-antigen complex of the present invention can be administered in any conventional way for treating an individual with a drug or antigen such as by injecting a solution containing the complex into the individual, ingestion of a solution containing the complex, having the individual swallow a pill containing the complex, etc. Where the allergic disease causes a skin reaction, the complex of the present invention can be administered topically by applying a complex containing solution to the surface of an individual's skin. The complex can be also administered through inhaling a misted solution containing the complex. The complex can be administered either proactively to a healthy individual to prevent the individual from having an allergic reaction of reactively to reduce the effects of an existing allergic reaction in an individual.

One preferred method of synthesizing the heat shock protein-antigen complexes of the present invention involves using a denatured protein column, as described above for the synthesis of heat shock protein-peptide complexes. In this method, HSPs (either bound or unbound to ADP) are bound to a denatured protein matrix or column such as a gelatin-agarose column. Then a complexing agent comprising an antigen is added to the denatured protein column to bind with the HSP complexes, thereby removing these complexes from the column and forming an HSP-antigen complex which is eluted.

One method for obtaining purified heat shock proteins for HSP-antigen complexes is to use a gelatin-agarose method such as that described in Nandan *et al*., "A rapid single-step purification method for immunogenic members of the HSP family; validation and application," in *Journal of Immunological Methods* (1994), 176:255-263. In this method of purification, cell lysates containing heat shock proteins are mixed with gelatin-agarose beads to bind the heat shock proteins to the beads. ATP is then added to the beads to release purified heat shock proteins. These purified HSPs can then be mixed with antigenic peptides in order to form complexes. In order to facilitate binding, ADP can also be added to the mixture.

Another method for releasing the complexes would be to add antigen complexing solution containing a antigen to the column to release the ADP-HSP complex as an ADP-HSP-antigen complex by binding to the antigen binding site on the HSP. When the antigen is a peptide, the antigen binds to the peptide binding site on the HSP.

Another preferred method of synthesizing heat shock protein-antigen complexes useful in the treatment method of the present invention involves adding heat shock proteins to an ADP matrix column to bind them to the matrix, adding a solution of peptides, polypeptides, denatured proteins or antigens to the column to bind them to the heat shock proteins as heat shock protein complexes and then adding a buffer containing ADP to the column to remove the ADP-heat shock protein-antigen complexes in an elution product.

Naturally occurring heat shock protein-antigen complexes can be purified for use in the treatment method of the present invention by adding a solution containing heat shock protein-antigen complexes to a column containing an ADP matrix to bind the heat shock proteins complexes to the ADP matrix and then adding a buffer containing ADP to the column remove the ADP-heat shock protein-antigen complexes in an elution product

In situations where it is undesirable to include ADP in the heat shock protein-antigen complex, heat shock proteins can be combined by is to mix HSPs and antigens in a container and let them sit for about 1 hour at about 37°C (See Roman *et al*., "Delayed-type hypersensitivity elicited by synthetic peptides complexed with mycobacterial tuberculosis hsp 70, *Immunology,* (1997) 90:52-56).

Although preferred methods of forming the heat shock protein-antigen complex used in the treatment method have been described above, other methods may be used to form the heat shock protein-antigen complex.

The antigens which are bound to the HSP carrier are typically obtained from purified cell lysates. The heat shock protein and antigen may be obtained from the same cell, from different cells in the same individual, from cells in different individuals and even from cells in different species. Also, either the heat shock protein or antigen can be formed synthetically, and the antigen may be recombinant or come from cell preps of allergy causing organisms.

The invention will now be described by way of example. The following example is illustrative and are not meant to limit the scope of the invention which is set forth by the appended claims.

### EXAMPLE 1

Heat shock proteins from cells or sub-cellular fractions are solubilized in lysis buffer (20 mM Tris-HCl pH 7.5, 0.15 M NaCl, 1% Triton X-100) containing a cocktail of protease inhibitors. This cocktail consists of 25 µg/ml of leupeptin, 25 µg/ml of E64 antipain and 0.5 mM (polymethylsulfonyl fluoride). The lysate is centrifuged at 10,000 x g for 20 min. at 4°C. to remove insoluble materials. The supernatant is incubated with appropriate amounts of gelatin-agarose beads by mixing for 2 hours at 25°C. This incubation will result in a gelatin-HSP(ADP)-peptide complex. The beads are collected by centrifugation and washed three times with a buffer adjusted to 0.5M NaCl, and once with 5mM Hepes, pH 7.5. Bound heat shock proteins are released by incubating the gelatin agarose beads with a solution containing a peptide or peptides representing the tumor or viral antigen. The heat shock protein-peptide complexes can then be used as a vaccine. Examples of suitable peptides that can be bound to heat shock proteins includes those found in Table 1 of Figures 1A to 1E.

### EXAMPLE 2

250 µg of synthesized Fel dI peptides are combined with 250 µg of purified mouse HSP70 for a total of 500 µg of HSP-peptide complex. Five B6D2F₁ mice are injected subcutaneously with 50 µg of HSP-peptide complex 10 days and 5 days prior to antigen challenge. Five control mice are also injected with saline on the same days. All ten mice are challenged with antigen by injecting 100 µg of peptide in 0.2 mls of complete Freund's adjuvant in four sites at the base of the tail and in both thighs.

### EXAMPLE 3

100 cat sensitive patients are randomly divided into treatment or placebo groups. Treatment groups are given an subcutaneous injection of 7.5 µg of HSP70-peptide complex. The injections occur once a week for 4 weeks.

Although the present invention has been fully described in conjunction with the preferred embodiment thereof with reference to the accompanying drawings, it is to be understood that various changes and modifications may be apparent to those skilled in the art. Such changes and modifications are to be understood as included within the scope of the present invention as defined by the appended claims, unless they depart therefrom.

## Claims

1. A method for synthesizing heat shock protein complexes comprising the steps of:
adding a heat shock protein to a denatured protein matrix to bind the heat shock protein to the matrix; and
adding a complexing solution comprising a peptide to elute a heat shock protein-peptide complex.

2. The method of claim 1, wherein said heat shock protein added to the matrix is complexed with ADP.

3. The method of claim 2, wherein said eluted heat shock protein-peptide complex comprises an ADP-heat shock protein peptide complex.

4. The method of claim 1, wherein said heat shock protein is added to the matrix as part of a solution containing said heat shock protein and said method further comprises the step of adding ADP to said heat shock protein-containing solution.

5. The method of claim 4, wherein ADP is added to said heat shock protein-containing solution prior to said heat shock protein-containing solution being added to the matrix.

6. The method of claim 4. wherein ADP is added to said heat shock protein-containing solution after said heat shock protein containing solution is added to the matrix.

7. The method of claim 4, wherein ADP is added to said heat shock protein-containing solution at the same time as said heat shock protein-containing solution is added to the matrix.

8. The method of claim 1, wherein said heat shock protein is added to the matrix as part of a heat shock protein-containing solution which includes ATP and the method further comprises the step of converting ATP in the heat shock protein-containing solution to ADP.

9. The method of claim 1, wherein said heat shock protein is added to the matrix as part of a cell lysate.

10. The method of claim 1 wherein the heat shock protein is selected from the group consisting of: hsp60. hsp65. rubisco binding protein and TCP-1 from eukaryotes: and GroEL/GroES, Mif4, TCPalpha and TCPbeta from yeast.

11. The method or claim 1 wherein the heat shock protein is selected from the group consisting of: hsp104, hsp105 and hsp110.

12. The method of claim 1 wherein the heat shock protein is selected from the group consisting of: DnaK proteins from prokaryotes; Ssa, Ssb, and Ssc from yeast; and hsp70, Grp75 and Grp78(Bip) from eukaryotes.

13. The method of claim 1 wherein the heat shock protein comprises one of the group consisting of: hsp90, gp96 and grp94.

14. The method of claim 1 wherein the peptide is from the same individual as the heat shock protein.

15. The method of claim 1 wherein the peptide and the heat shock protein are from different individuals.

16. The method of claim 1 wherein the peptide and the heat shock protein are from different species.

17. The method of claim 1 wherein the complexing solution comprises a cell lysate.

18. An apparatus for synthesizing heat shock protein-peptide complexes comprising a heat shock protein bound to a denatured protein matrix, said heat shock protein being complexed with ADP.

19. The apparatus of claim 18 wherein said denatured protein matrix comprises a denatured protein column.

20. The apparatus of claim 18 wherein the heat shock protein is selected from the group consisting of: hsp60. hsp65, rubisco binding protein and TCP-1 from eukaryotes: and GroEL/GroES. Mif4. TCPalpha and TCPbeta from yeast.

21. The apparatus of claim 18 wherein the heat shock protein is selected from the group consisting of: hsp104, hsp105 and hsp 110.

22. The apparatus of claim 18 wherein the heat shock protein is selected from the group consisting of: DnaK proteins from prokaryotes; Ssa, Ssb, and Ssc from yeast; and hsp70, Grp75 and Grp78(Bip) from eukaryotes.

23. The apparatus of claim 18 wherein the heat shock protein is selected from the group consisting of: hsp90. gp96 and grp94.

## Patentansprüche

1. Verfahren zur Synthese von Hitzeschock-Protein-Komplexen, folgende Schritte aufweisend:
Zugeben eines Hitzeschock-Proteins zu einer denaturierten Proteinmatrix, um das Hitzeschock-Protein an die Matrix zu binden, und
Zugeben einer komplexierenden Lösung, die ein Peptid aufweist, um einen Hitzeschock-Protein-Peptid-Komplex herauszulösen.

2. Verfahren nach Anspruch 1, bei dem das zu der Matrix zugegebene Hitzeschock-Protein mit ADP komplexiert wird.

3. Verfahren nach Anspruch 2, bei dem der herausgelöste Hitzeschock-Protein-Peptid-Komplex einen ADP-Hitzeschock-Protein-Peptid-Komplex aufweist.

4. Verfahren nach Anspruch 1, bei dem das Hitzeschock-Protein als Teil einer Lösung, die das Hitzeschock-Protein enthält, zu der Matrix zugegeben wird, und das Verfahren außerdem den Schritt des Zugebens von ADP zu der Hitzeschock-Protein enthaltenden Lösung aufweist.

5. Verfahren nach Anspruch 4, bei dem ADP zu der Hitzeschock-Protein enthaltenden Lösung zugegeben wird bevor die Hitzeschock-Protein enthaltende Lösung zu der Matrix zugegeben wird.

6. Verfahren nach Anspruch 4, bei dem ADP zu der Hitzeschock-Protein enthaltenden Lösung zugegeben wird nachdem die Hitzeschock-Protein enthaltende Lösung zu der Matrix zugegeben wurde.

7. Verfahren nach Anspruch 4, bei dem ADP zu der Hitzeschock-Protein enthaltenden Lösung zur gleichen Zeit zugegeben wird, zu der die Hitzeschock-Protein enthaltende Lösung zu der Matrix zugegeben wird.

8. Verfahren nach Anspruch 1, bei dem das Hitzeschock-Protein als Teil einer Hitzeschock-Protein enthaltenden Lösung, die ATP aufweist, zu der Matrix zugegeben wird, und das Verfahren außerdem den Schritt des Umwandelns von ATP in der Hitzeschock-Protein enthaltenden Lösung zu ADP aufweist.

9. Verfahren nach Anspruch 1, bei dem das Hitzeschock-Protein als Teil eines Zell-Lysats zu der Matrix zugegeben wird.

10. Verfahren nach Anspruch 1, bei dem das Hitzeschock-Protein ausgewählt wird aus der Gruppe, die besteht aus hsp60, hsp 65, Rubisco-Bindungsprotein und TCP-1 von Eukaryonten, und GroEL/GroES, Mif4, TCPalpha und TCPbeta von Hefe.

11. Verfahren nach Anspruch 1, bei dem das Hitzeschock-Protein ausgewählt wird aus der Gruppe, die besteht aus: hsp104, hsp105 und hsp110.

12. Verfahren nach Anspruch 1, bei dem das Hitzeschock-Protein ausgewählt wird aus der Gruppe, die besteht aus: DnaK-Proteinen von Prokaryonten; Ssa, Ssb und Ssc von Hefe, und hsp70, Grp75 und Grp78(Bip) von Eukaryonten.

13. Verfahren nach Anspruch 1, bei dem das Hitzeschock-Protein eines aufweist aus der Gruppe, die besteht aus: hsp90, gp96 und grp94.

14. Verfahren nach Anspruch 1, bei dem das Peptid von demselben Individuum stammt wie das Hitzeschock-Protein.

15. Verfahren nach Anspruch 1, bei dem das Peptid und das Hitzeschock-Protein von verschiedenen Individuen sind.

16. Verfahren nach Anspruch 1, bei dem das Peptid und das Hitzeschock-Protein von verschiedenen Spezies sind.

17. Verfahren nach Anspruch 1, bei dem die komplexierende Lösung ein Zell-Lysat aufweist.

18. Vorrichtung zur Synthese von Hitzeschock-Protein-Peptid-Komplexen, aufweisend ein Hitzeschock-Protein, das an eine denaturierte Proteinmatrix gebunden ist, wobei das Hitzeschock-Protein mit ADP komplexiert ist.

19. Vorrichtung nach Anspruch 18, bei der die denaturierte Proteinmatrix eine denaturierte Protein-Säule aufweist.

20. Vorrichtung nach Anspruch 18, bei der das Hitzeschock-Protein ausgewählt ist aus der Gruppe, die besteht aus: hsp60, hsp65, Rubisco-Bindungsprotein und TCP-1 von Eukaryonten, und GroEL/GroES, Mif4, TCPalpha und TCPbeta von Hefe.

21. Vorrichtung nach Anspruch 18, bei der das Hitzeschock-Protein ausgewählt ist aus der Gruppe, die besteht aus: hsp104, hsp105 und hsp110.

22. Vorrichtung nach Anspruch 18, bei der das Hitzeschock-Protein ausgewählt ist aus der Gruppe, die besteht aus: DnaK-Proteinen von Prokaryonten, Ssa, Ssb und Ssc von Hefe, und hsp70, Grp75 und Grp78(Bip) von Eukaryonten.

23. Vorrichtung nach Anspruch 18, bei der das Hitzeschock-Protein ausgewählt ist aus der Gruppe, die besteht aus: hsp90, gp96 und grp94.

## Revendications

1. Procédé pour synthétiser des complexes à base de protéine de choc thermique, comprenant les étapes de :
addition d'une protéine de choc thermique à une matrice de protéine dénaturée pour lier la protéine de choc thermique à la matrice ; et
addition d'une solution de complexation comprenant un peptide pour éluer un complexe de protéine de choc thermique-peptide.

2. Procédé selon la revendication 1, dans lequel ladite protéine de choc thermique ajoutée à la matrice est complexée avec du ADP.

3. Procédé selon la revendication 2, dans lequel ledit complexe élué de protéine de choc thermique-peptide comprend un complexe peptidique de ADP-protéine de choc thermique.

4. Procédé selon la revendication 1, dans lequel ladite protéine de choc thermique est ajoutée à la matrice en tant que partie d'une solution contenant ladite protéine de choc thermique, et ledit procédé comprend, en outre, l'étape d'addition de ADP à ladite solution contenant la protéine de choc thermique.

5. Procédé selon la revendication 4, dans lequel le ADP est ajouté à ladite solution contenant la protéine de choc thermique avant que ladite solution contenant la protéine de choc thermique soit ajoutée à la matrice.

6. Procédé selon la revendication 4, dans lequel le ADP est ajouté à ladite solution contenant la protéine de choc thermique après que ladite solution contenant la protéine de choc thermique a été ajoutée à la matrice.

7. Procédé selon la revendication 4, dans lequel le ADP est ajouté à ladite solution contenant la protéine de choc thermique au même moment où ladite solution contenant la protéine de choc thermique est ajoutée à la matrice.

8. Procédé selon la revendication 1, dans lequel ladite protéine de choc thermique est ajoutée à la matrice en tant que partie d'une solution contenant la protéine de choc thermique qui comprend du ATP, et le procédé comprend, en outre, l'étape de transformation du ATP dans la solution contenant la protéine de choc thermique en ADP.

9. Procédé selon la revendication 1, dans lequel ladite protéine de choc thermique est ajoutée à la matrice en tant que partie d'un lysat de cellules.

10. Procédé selon la revendication 1, dans lequel la protéine de choc thermique est choisie dans l'ensemble constitué par : hsp60, hsp65, protéine de liaison de rubisco et TCP-1 provenant de cellules eucaryotes ; et GroEL/GroES, Mif4, TCP-alpha et TCP-bêta provenant d'une levure.

11. Procédé selon la revendication 1, dans lequel la protéine de choc thermique est choisie dans l'ensemble constitué par : hsp104, hsp105 et hsp110.

12. Procédé selon la revendication 1, dans lequel la protéine de choc thermique est choisie dans l'ensemble constitué par : protéines DnaK de procaryotes ; Ssa, Ssb et Ssc de levure ; et hsp70, Grp75 et Grp78(Bip) d'eucaryotes.

13. Procédé selon la revendication 1, dans lequel la protéine de choc thermique comprend l'une de l'ensemble constitué par : hsp90, gp96 et grp94.

14. Procédé selon la revendication 1, dans lequel le peptide provient du même individu que la protéine de choc thermique.

15. Procédé selon la revendication 1, dans lequel le peptide et la protéine de choc thermique proviennent d'individus différents.

16. Procédé selon la revendication 1, dans lequel le peptide et la protéine de choc thermique proviennent d'espèces différentes.

17. Procédé selon la revendication 1, dans lequel la solution de complexation comprend un lysat de cellules.

18. Appareil pour synthétiser des complexes de protéine de choc thermique-peptide comprenant une protéine de choc thermique liée à une matrice de protéine dénaturée, ladite protéine de choc thermique étant complexée avec du ADP.

19. Appareil selon la revendication 18, dans lequel ladite matrice de protéine dénaturée comprend une colonne de protéine dénaturée.

20. Appareil selon la revendication 18, dans lequel la protéine de choc thermique est choisie dans l'ensemble constitué par : hsp60, hsp65, protéine de liaison de rubisco et TCP-1 provenant d'eucaryotes ; et GroEL/GroES, Mif4, TCP-alpha et TCP-bêta provenant d'une levure.

21. Appareil selon la revendication 18, dans lequel la protéine de choc thermique est choisie dans l'ensemble constitué par : hsp104, hsp105 et hsp110.

22. Appareil selon la revendication 18, dans lequel la protéine de choc thermique est choisie dans l'ensemble constitué par : protéines DnaK de procaryotes ; Ssa, Ssb et Ssc de levure ; et hsp70, Grp75 et Grp78(Bip) d'eucaryotes.

23. Appareil selon la revendication 18, dans lequel la protéine de choc thermique est choisie dans l'ensemble constitué par : hsp90, gp96 et grp94.


### SEQUENCE LISTING

<110> University of New Mexico
<120> Heat Shock Protein Complexes
<130> K51401/7
<140> 98 961 905.1
   <141> 1998-12-04
<150> US 08/985,548
   <151> 1997-12-05
<150> US 08/986,234
   <151> 1997-12-05
<160> 114
<170> PatentIn version 3.0
<210> 1
   <211> 9
   <212> PRT
   <213> Human
<400> 1
   Glu Ala Asp Pro Thr Gly His Ser Tyr
   1               5
<210> 2
   <211> 17
   <212> PRT
   <213> Feline immunodeficiency virus
<400> 2
<210> 3
   <211> 10
   <212> PRT
   <213> Feline immunodeficiency virus
<400> 3
   Arg Ala Ile Ser Ser Trp Lys Gln Arg Asn
   1 5 10
<210> 4
   <211> 10
   <212> PRT
   <213> Feline immunodeficiency virus
<400> 4
   Ser Trp Lys Gln Arg Asn Arg Trp Glu Trp
   1 5 10
<210> 5
   <211> 10
   <212> PRT
   <213> Feline immunodeficiency virus
<400> 5
   Gln Arg Asn Arg Trp Glu Trp Arg Pro Asp
   1 5 10
<210> 6
   <211> 19
   <212> PRT
   <213> Foot-and-mouth disease virus
<400> 6
<210> 7
   <211> 15
   <212> PRT
   <213> Foot-and-mouth disease virus
<400> 7
   Pro Phe Gly His Leu Thr Lys Leu Glu Leu Pro Thr Asp His His
   1 5 10 15
<210> 8
   <211> 15
   <212> PRT
   <213> Foot-and-mouth disease virus
<400> 8
   Ser Ile Ile Asn Asn Tyr Tyr Met Gln Gln Tyr Gln Asn Ser Met
   1 5 10 15
<210> 9
   <211> 35
   <212> PRT
   <213> Human
<400> 9
<210> 10
   <211> 14
   <212> PRT
   <213> Human
<400> 10
   Lys Met Phe Tyr Gln Leu Ala Lys Thr Cys Pro Val Gln Leu
   1 5 10
<210> 11
   <211> 9
   <212> PRT
   <213> Human
<400> 11
   Phe Tyr Gln Leu Ala Thr Cys Pro Val
   1 5
<210> 12
   <211> 10
   <212> PRT
   <213> Human
<400> 12
   Phe Leu Gln Leu Ala Lys Thr Cys Pro Val
   1 5 10
<210> 13
   <211> 15
   <212> PRT
   <213> Human immunodeficiency virus
<400> 13
   Arg Ile Gln Arg Gly Pro Gly Arg Ala Phe Val Thr Ile Gly Lys
   1 5 10 15
<210> 14
   <211> 15
   <212> PRT
   <213> Human immunodeficiency virus
<400> 14
   Arg Ile His Ile Gly Pro Gly Arg Ala Phe Tyr Thr Thr Lys Asn
   1 5 10 15
<210> 15
   <211> 15
   <212> PRT
   <213> Human immunodeficiency virus
<400> 15
   Ser Ile Thr Lys Gly Pro Gly Arg Val Ile Tyr Ala Thr Gly Gln
   1 5 10 15
<210> 16
   <211> 15
   <212> PRT
   <213> Human immunodeficiency virus
<400> 16
   Thr Leu His Met Gly Pro Lys Arg Ala Phe Tyr Ala Thr Gly Asn
   1 5 10 15
<210> 17
   <211> 15
   <212> PRT
   <213> Human immunodeficiency virus
<400> 17
   Ser Ile His Ile Gly Pro Gly Arg Ala Phe Tyr Thr Thr Gly Glu
   1 5 10 15
<210> 18
   <211> 15
   <212> PRT
   <213> Human immunodeficiency virus
<400> 18
   Arg Ile His Ile Gly Pro Gly Arg Ala Phe Tyr Thr Thr Lys Asn
   1 5 10 15
<210> 19
   <211> 15
   <212> PRT
   <213> Human immunodeficiency virus
<400> 19
   Ser Ile Arg Ile Gly Pro Gly Lys Val Phe Thr Ala Lys Gly Gly
   1 5 10 15
<210> 20
   <211> 15
   <212> PRT
   <213> Human immunodeficiency virus
<400> 20
   Ser Ile Ser Ile Gly Pro Gly Arg Ala Phe Phe Ala Thr Thr Asp
   1 5 10 15
<210> 21
   <211> 15
   <212> PRT
   <213> Human immunodeficiency virus
<400> 21
   Arg Ile His Ile Gly Pro Gly Arg Ala Phe Tyr Thr Thr Lys Asn
   1 5 10 15
<210> 22
   <211> 15
   <212> PRT
   <213> Human immunodeficiency virus
<400> 22
   Ser Leu Ser Ile Gly Pro Gly Arg Ala Phe Arg Thr Arg Glu Ile
   1 5 10 15
<210> 23
   <211> 15
   <212> PRT
   <213> Human immunodeficiency virus
<400> 23
   Gly Ile Ala Ile Gly Pro Gly Arg Thr Leu Tyr Ala Arg Glu Lys
   1 5 10 15
<210> 24
   <211> 15
   <212> PRT
   <213> Human immunodeficiency virus
<400> 24
   Ser Ile Thr Lys Gly Pro Gly Arg Val Ile Tyr Ala Thr Gly Gln
   1 5 10 15
<210> 25
   <211> 15
   <212> PRT
   <213> Human immunodeficiency virus
<400> 25
   Arg Val Thr Leu Gly Pro Gly Arg Val Trp Tyr Thr Thr Gly Glu
   1 5 10 15
<210> 26
   <211> 15
   <212> PRT
   <213> Human immunodeficiency virus
<400> 26
   Ser Ile His Ile Gly Pro Gly Arg Ala Phe Tyr Ala Thr Gly Asp
   1 5 10 15
<210> 27
   <211> 15
   <212> PRT
   <213> Human immunodeficiency virus
<400> 27
   Ser Ile Tyr Ile Gly Pro Gly Arg Ala Phe His Thr Thr Gly Arg
   1 5 10 15
<210> 28
   <211> 15
   <212> PRT
   <213> Human immunodeficiency virus
<400> 28
   Arg Ile Gln Arg Gly Pro Gly Arg Ala Phe Val Thr Ile Gly Lys
   1 5 10 15
<210> 29
   <211> 15
   <212> PRT
   <213> Human immunodeficiency virus
<400> 29
   Gly Ile His Phe Gly Pro Gly Gln Ala Leu Tyr Thr Thr Gly Ile
   1               5                   10                  15
<210> 30
   <211> 15
   <212> PRT
   <213> Human immunodeficiency virus
<400> 30
   Ser Thr Pro Ile Gly Leu Gly Gln Ala Leu Tyr Thr Thr Arg Gly
   1               5                   10                  15
<210> 31
   <211> 15
   <212> PRT
   <213> Human immunodeficiency virus
<400> 31
   Ser Thr Pro Ile Gly Leu Gly Gln Ala Leu Tyr Thr Thr Arg Ile
   1               5                   10                  15
<210> 32
   <211> 15
   <212> PRT
   <213> Human immunodeficiency virus
<400> 32
   Arg Thr Pro Thr Gly Leu Gly Gln Ser Leu Tyr Thr Thr Arg Ser
   1               5                   10                  15
<210> 33
   <211> 16
   <212> PRT
   <213> Hepatitis C virus
<400> 33
<210> 34
   <211> 16
   <212> PRT
   <213> Hepatitis C virus
<400> 34
<210> 35
   <211> 10
   <212> PRT
   <213> Hepatitis C virus
<400> 35
   Leu Met Gly Tyr Ile Pro Leu Val Gly Ala
   1 5 10
<210> 36
   <211> 17
   <212> PRT
   <213> Human
<220>
   <221> X
   <222> (7)..(9)
   <223> Xaa
<400> 36
<210> 37
   <211> 12
   <212> PRT
   <213> Chlamydia trachomatis
<400> 37
   Thr Ile Asn Lys Pro Lys Gly Tyr Thr Gly Lys Glu
   1 5 10
<210> 38
   <211> 9
   <212> PRT
   <213> Human immunodeficiency virus
<400> 38
   His Ile Val Trp Ala Ser Arg Phe Leu
   1 5
<210> 39
   <211> 9
   <212> PRT
   <213> Human immunodeficiency virus
<400> 39
   Ile Leu Gly Gln Leu Gln Pro Ser Leu
   1 5
<210> 40
   <211> 9
   <212> PRT
   <213> Human immunodeficiency virus
<400> 40
   Ser Leu Gln Thr Gly Ser Glu Glu Leu
   1 5
<210> 41
   <211> 9
   <212> PRT
   <213> Human immunodeficiency virus
<400> 41
   Glu Leu Arg Ser Leu Tyr Asn Thr Val
   1 5
<210> 42
   <211> 9
   <212> PRT
   <213> Human immunodeficiency virus
<400> 42
   Ser Leu Tyr Asn Thr Val Ala Thr Leu
   1 5
<210> 43
   <211> 9
   <212> PRT
   <213> Human immunodeficiency virus
<400> 43
   Glu Ile Lys Asp Thr Lys Glu Ala Leu
   1 5
<210> 44
   <211> 9
   <212> PRT
   <213> Human immunodeficiency virus
<400> 44
   Thr Leu Asn Ala Trp Val Lys Val Val
   1 5
<210> 45
   <211> 9
   <212> PRT
   <213> Human immunodeficiency virus
<400> 45
   Asp Leu Asn Thr Met Leu Asn Thr Val
   1 5
<210> 46
   <211> 9
   <212> PRT
   <213> Human immunodeficiency virus
<400> 46
   Asp Ile Ala Gly Thr Thr Ser Thr Leu
   1               5
<210> 47
   <211> 9
   <212> PRT
   <213> Human immunodeficiency virus
<400> 47
   Thr Leu Gln Glu Gln Ile Gly Trp Met
   1               5
<210> 48
   <211> 9
   <212> PRT
   <213> Human immunodeficiency virus
<400> 48
   Glu Ile Tyr Lys Arg Trp Ile Leu Leu
   1               5
<210> 49
   <211> 9
   <212> PRT
   <213> Human immunodeficiency virus
<400> 49
   Ile Ile Leu Gly Leu Asn Lys Ile Val
   1               5
<210> 50
   <211> 8
   <212> PRT
   <213> Human immunodeficiency virus
<400> 50
   Phe Met Met Thr Ala Cys Gly Val
   1               5
<210> 51
   <211> 9
   <212> PRT
   <213> Human immunodeficiency virus
<400> 51
   Val Leu Ala Glu Ala Met Ser Gln Val
   1 5
<210> 52
   <211> 9
   <212> PRT
   <213> Human immunodeficiency virus
<400> 52
   Pro Ile Asp Lys Glu Leu Tyr Pro Leu
   1 5
<210> 53
   <211> 9
   <212> PRT
   <213> Human immunodeficiency virus
<400> 53
   Leu Leu Thr Gln Ile Gly Cys Thr Leu
   1 5
<210> 54
   <211> 9
   <212> PRT
   <213> Human immunodeficiency virus
<400> 54
   Pro Ile Glu Thr Val Pro Val Lys Leu
   1 5
<210> 55
   <211> 9
   <212> PRT
   <213> Human immunodeficiency virus
<400> 55
   Ala Leu Val Glu Ile Cys Thr Glu Met
   1 5
<210> 56
   <211> 9
   <212> PRT
   <213> Human immunodeficiency virus
<400> 56
   Gly Ile Arg Tyr Gln Tyr Asn Val Leu
   1 5
<210> 57
   <211> 9
   <212> PRT
   <213> Human immunodeficiency virus
<400> 57
   Tyr Ile Tyr Gln Tyr Met Asp Asp Leu
   1 5
<210> 58
   <211> 9
   <212> PRT
   <213> Human immunodeficiency virus
<400> 58
   Lys Ile Glu Glu Leu Arg Gln His Leu
   1 5
<210> 59
   <211> 9
   <212> PRT
   <213> Human immunodeficiency virus
<400> 59
   Glu Leu His Pro Asp Lys Trp Thr Val
   1 5
<210> 60
   <211> 9
   <212> PRT
   <213> Human immunodeficiency virus
<400> 60
   Asp Ile Gln Lys Leu Val Gly Lys Leu
   1 5
<210> 61
   <211> 9
   <212> PRT
   <213> Human immunodeficiency virus
<400> 61
   Lys Leu Lys Arg Gly Thr Lys Ala Leu
   1 5
<210> 62
   <211> 9
   <212> PRT
   <213> Human immunodeficiency virus
<400> 62
   Glu Leu Ala Glu Asn Arg Glu Ile Leu
   1 5
<210> 63
   <211> 9
   <212> PRT
   <213> Human immunodeficiency virus
<400> 63
   Ile Leu Lys Glu Pro Val His Gly Val
   1 5
<210> 64
   <211> 8
   <212> PRT
   <213> Human immunodeficiency virus
<400> 64
   Pro Leu Val Lys Leu Trp Tyr Leu
   1 5
<210> 65
   <211> 9
   <212> PRT
   <213> Human immunodeficiency virus
<400> 65
   Glu Leu Gln Ala Ile Tyr Leu Ala Leu
   1               5
<210> 66
   <211> 9
   <212> PRT
   <213> Human immunodeficiency virus
<400> 66
   Tyr Leu Ala Leu Gln Asp Ser Gly Leu
   1               5
<210> 67
   <211> 9
   <212> PRT
   <213> Human immunodeficiency virus
<400> 67
   Ala Leu Gln Asp Ser Gly Leu Glu Val
   1               5
<210> 68
   <211> 9
   <212> PRT
   <213> Human immunodeficiency virus
<400> 68
   Leu Ile Lys Lys Glu Lys Val Tyr Leu
   1               5
<210> 69
   <211> 9
   <212> PRT
   <213> Human immunodeficiency virus
<400> 69
   His Leu Glu Gly Lys Val Ile Leu Val
   1               5
<210> 70
   <211> 9
   <212> PRT
   <213> Human immunodeficiency virus
<400> 70
   Glu Leu Lys Lys Ile Ile Gly Gln Val
   1               5
<210> 71
   <211> 9
   <212> PRT
   <213> Human immunodeficiency virus
<400> 71
   Pro Leu Trp Lys Gly Pro Ala Lys Leu
   1               5
<210> 72
   <211> 9
   <212> PRT
   <213> Human immunodeficiency virus
<400> 72
   Leu Leu Trp Lys Gly Glu Gly Ala Val
   1               5
<210> 73
   <211> 9
   <212> PRT
   <213> Human immunodeficiency virus
<400> 73
   Ile Ile Arg Asp Tyr Gly Lys Gln Met
   1               5
<210> 74
   <211> 9
   <212> PRT
   <213> Human immunodeficiency virus
<400> 74
   Met Ile Val Trp Gln Val Asp Arg Met
   1 5
<210> 75
   <211> 9
   <212> PRT
   <213> Human immunodeficiency virus
<400> 75
   Arg Ile Arg Thr Trp Lys Ser Leu Val
   1 5
<210> 76
   <211> 9
   <212> PRT
   <213> Human immunodeficiency virus
<400> 76
   Asp Leu Ala Asp Gln Leu Ile His Leu
   1 5
<210> 77
   <211> 9
   <212> PRT
   <213> Human immunodeficiency virus
<400> 77
   Lys Ile Lys Pro Pro Leu Pro Ser Val
   1 5
<210> 78
   <211> 9
   <212> PRT
   <213> Human immunodeficiency virus
<400> 78
   Met Ile Cys Ser Ala Thr Glu Lys Leu
   1 5
<210> 79
   <211> 9
   <212> PRT
   <213> Human immunodeficiency virus
<400> 79
   Ile Ile Leu Ser Trp Asp Gln Ser Leu
   1               5
<210> 80
   <211> 9
   <212> PRT
   <213> Human immunodeficiency virus
<400> 80
   Lys Leu Thr Ser Leu Cys Val Ser Leu
   1               5
<210> 81
   <211> 9
   <212> PRT
   <213> Human immunodeficiency virus
<400> 81
   Lys Leu Thr Ser Cys Asn Thr Ser Val
   1               5
<210> 82
   <211> 9
   <212> PRT
   <213> Human immunodeficiency virus
<400> 82
   Val Ile Thr Gln Ala Cys Pro Lys Val
   1               5
<210> 83
   <211> 9
   <212> PRT
   <213> Human immunodeficiency virus
<400> 83
   Ile Ile Val Gln Leu Asn Thr Ser Val
   1               5
<210> 84
   <211> 9
   <212> PRT
   <213> Human immunodeficiency virus
<400> 84
   Thr Leu Lys Gln Ile Ala Ser Lys Leu
   1 5
<210> 85
   <211> 9
   <212> PRT
   <213> Human immunodeficiency virus
<400> 85
   Ala Ile Ile Asn Met Trp Gln Lys Val
   1 5
<210> 86
   <211> 9
   <212> PRT
   <213> Human immunodeficiency virus
<400> 86
   Phe Leu Gly Ala Ala Gly Ser Thr Met
   1 5
<210> 87
   <211> 9
   <212> PRT
   <213> Human immunodeficiency virus
<400> 87
   Thr Leu Thr Val Gln Ala Arg Gln Leu
   1 5
<210> 88
   <211> 9
   <212> PRT
   <213> Human immunodeficiency virus
<400> 88
   Gly Ile Val Gln Gln Gln Asn Asn Leu
   1               5
<210> 89
   <211> 9
   <212> PRT
   <213> Human immunodeficiency virus
<400> 89
   Ala Ile Glu Ala Gln Gln His Leu Leu
   1               5
<210> 90
   <211> 9
   <212> PRT
   <213> Human immunodeficiency virus
<400> 90
   Gln Leu Gln Ala Arg Ile Leu Ala Val
   1               5
<210> 91
   <211> 9
   <212> PRT
   <213> Human immunodeficiency virus
<400> 91
   Arg Ile Leu Ala Val Glu Arg Tyr Leu
   1               5
<210> 92
   <211> 9
   <212> PRT
   <213> Human immunodeficiency virus
<400> 92
   Gly Ile Trp Gly Cys Ser Gly Lys Leu
   1               5
<210> 93
   <211> 9
   <212> PRT
   <213> Human immunodeficiency virus
<400> 93
   Gln Ile Trp Asn His Thr Thr Trp Met
   1 5
<210> 94
   <211> 9
   <212> PRT
   <213> Human immunodeficiency virus
<400> 94
   Tyr Ile Lys Leu Phe Ile Met Ile Val
   1 5
<210> 95
   <211> 9
   <212> PRT
   <213> Human immunodeficiency virus
<400> 95
   Phe Ile Met Ile Val Gly Gly Leu Val
   1 5
<210> 96
   <211> 9
   <212> PRT
   <213> Human immunodeficiency virus
<400> 96
   Met Ile Val Gly Gly Leu Val Gly Leu
   1 5
<210> 97
   <211> 9
   <212> PRT
   <213> Human immunodeficiency virus
<400> 97
   Gly Leu Arg Ile Val Phe Ala Val Leu
   1 5
<210> 98
   <211> 9
   <212> PRT
   <213> Human immunodeficiency virus
<400> 98
   Ser Ile Arg Leu Val Asn Gly Ser Leu
   1 5
<210> 99
   <211> 9
   <212> PRT
   <213> Human immunodeficiency virus
<400> 99
   Arg Leu Val Asn Gly Ser Leu Ala Leu
   1 5
<210> 100
   <211> 9
   <212> PRT
   <213> Human immunodeficiency virus
<400> 100
   Ser Leu Ala Leu Ile Trp Asp Asp Leu
   1 5
<210> 101
   <211> 9
   <212> PRT
   <213> Human immunodeficiency virus
<400> 101
   Leu Ile Trp Asp Asp Leu Arg Ser Leu
   1 5
<210> 102
   <211> 9
   <212> PRT
   <213> Human immunodeficiency virus
<400> 102
   Arg Leu Arg Asp Leu Leu Leu Ile Val
   1 5
<210> 103
   <211> 9
   <212> PRT
   <213> Human immunodeficiency virus
<400> 103
   Leu Leu Leu Ile Val Thr Arg Ile Val
   1 5
<210> 104
   <211> 9
   <212> PRT
   <213> Human immunodeficiency virus
<400> 104
   Leu Ile Val Thr Arg Ile Val Glu Leu
   1 5
<210> 105
   <211> 9
   <212> PRT
   <213> Human immunodeficiency virus
<400> 105
   Leu Leu Gln Tyr Trp Ser Gln Glu Leu
   1 5
<210> 106
   <211> 9
   <212> PRT
   <213> Human immunodeficiency virus
<400> 106
   Glu Leu Lys Asn Ser Ala Val Ser Leu
   1 5
<210> 107
   <211> 9
   <212> PRT
   <213> Human immunodeficiency virus
<400> 107
   Leu Leu Asn Ala Thr Ala Ile Ala Val
   1 5
<210> 108
   <211> 9
   <212> PRT
   <213> Human immunodeficiency virus
<400> 108
   Ala Ile Ile Arg Ile Leu Gln Gln Leu
   1 5
<210> 109
   <211> 9
   <212> PRT
   <213> Human immunodeficiency virus
<400> 109
   Ile Ile Arg Ile Leu Gln Gln Leu Leu
   1 5
<210> 110
   <211> 9
   <212> PRT
   <213> Human immunodeficiency virus
<400> 110
   Tyr Leu Gly Arg Ser Ala Glu Pro Val
   1 5
<210> 111
   <211> 9
   <212> PRT
   <213> Human immunodeficiency virus
<400> 111
   Gln Ile Leu Val Glu Ser Pro Thr Val
   1 5
<210> 112
   <211> 9
   <212> PRT
   <213> Human immunodeficiency virus
<400> 112
   Ile Leu Val Glu Ser Pro Thr Val Leu
   1               5
<210> 113
   <211> 9
   <212> PRT
   <213> Human immunodeficiency virus
<400> 113
   Gln Ile Ala Ile Val Ala Ala Leu Val
   1               5
<210> 114
   <211> 9
   <212> PRT
   <213> Human immunodeficiency virus
<400> 114
   Gly Met Asp Asp Pro Glu Lys Glu Val
   1               5

### SEQUENCE LISTING

<110> University of New Mexico
<120> Heat Shock Protein Complexes
<130> K51401/7
<140> 98 961 905.1
   <141> 1998-12-04
<150> US 08/985,548
   <151> 1997-12-05
<150> US 08/986,234
   <151> 1997-12-05
<160> 114
<170> PatentIn version 3.0
<210> 1
   <211> 9
   <212> PRT
   <213> Human
<400> 1
   Glu Ala Asp Pro Thr Gly His Ser Tyr
   1 5
<210> 2
   <211> 17
   <212> PRT
   <213> Feline immunodeficiency virus
<400> 2
<210> 3
   <211> 10
   <212> PRT
   <213> Feline immunodeficiency virus
<400> 3
   Arg Ala Ile Ser Ser Trp Lys Gln Arg Asn
   1 5 10
<210> 4
   <211> 10
   <212> PRT
   <213> Feline immunodeficiency virus
<400> 4
   Ser Trp Lys Gln Arg Asn Arg Trp Glu Trp
   1 5 10
<210> 5
   <211> 10
   <212> PRT
   <213> Feline immunodeficiency virus
<400> 5
   Gln Arg Asn Arg Trp Glu Trp Arg Pro Asp
   1 5 10
<210> 6
   <211> 19
   <212> PRT
   <213> Foot-and-mouth disease virus
<400> 6
<210> 7
   <211> 15
   <212> PRT
   <213> Foot-and-mouth disease virus
<400> 7
   Pro Phe Gly His Leu Thr Lys Leu Glu Leu Pro Thr Asp His His
   1 5 10 15
<210> 8
   <211> 15
   <212> PRT
   <213> Foot-and-mouth disease virus
<400> 8
   Ser Ile Ile Asn Asn Tyr Tyr Met Gln Gln Tyr Gln Asn Ser Met
   1 5 10 15
<210> 9
   <211> 35
   <212> PRT
   <213> Human
<400> 9
<210> 10
   <211> 14
   <212> PRT
   <213> Human
<400> 10
   Lys Met Phe Tyr Gln Leu Ala Lys Thr Cys Pro Val Gln Leu
   1 5 10
<210> 11
   <211> 9
   <212> PRT
   <213> Human
<400> 11
   Phe Tyr Gln Leu Ala Thr Cys Pro Val
   1 5
<210> 12
   <211> 10
   <212> PRT
   <213> Human
<400> 12
   Phe Leu Gln Leu Ala Lys Thr Cys Pro Val
   1 5 10
<210> 13
   <211> 15
   <212> PRT
   <213> Human immunodeficiency virus
<400> 13
   Arg Ile Gln Arg Gly Pro Gly Arg Ala Phe Val Thr Ile Gly Lys
   1               5                   10                  15
<210> 14
   <211> 15
   <212> PRT
   <213> Human immunodeficiency virus
<400> 14
   Arg Ile His Ile Gly Pro Gly Arg Ala Phe Tyr Thr Thr Lys Asn
   1               5                   10                  15
<210> 15
   <211> 15
   <212> PRT
   <213> Human immunodeficiency virus
<400> 15
   Ser Ile Thr Lys Gly Pro Gly Arg Val Ile Tyr Ala Thr Gly Gln
   1               5                   10                  15
<210> 16
   <211> 15
   <212> PRT
   <213> Human immunodeficiency virus
<400> 16
   Thr Leu His Met Gly Pro Lys Arg Ala Phe Tyr Ala Thr Gly Asn
   1               5                   10                  15
<210> 17
   <211> 15
   <212> PRT
   <213> Human immunodeficiency virus
<400> 17
   Ser Ile His Ile Gly Pro Gly Arg Ala Phe Tyr Thr Thr Gly Glu
   1               5                   10                  15
<210> 18
   <211> 15
   <212> PRT
   <213> Human immunodeficiency virus
<400> 18
   Arg Ile His Ile Gly Pro Gly Arg Ala Phe Tyr Thr Thr Lys Asn
   1 5 10 15
<210> 19
   <211> 15
   <212> PRT
   <213> Human immunodeficiency virus
<400> 19
   Ser Ile Arg Ile Gly Pro Gly Lys Val Phe Thr Ala Lys Gly Gly
   1 5 10 15
<210> 20
   <211> 15
   <212> PRT
   <213> Human immunodeficiency virus
<400> 20
   Ser Ile Ser Ile Gly Pro Gly Arg Ala Phe Phe Ala Thr Thr Asp
   1 5 10 15
<210> 21
   <211> 15
   <212> PRT
   <213> Human immunodeficiency virus
<400> 21
   Arg Ile His Ile Gly Pro Gly Arg Ala Phe Tyr Thr Thr Lys Asn
   1 5 10 15
<210> 22
   <211> 15
   <212> PRT
   <213> Human immunodeficiency virus
<400> 22
   Ser Leu Ser Ile Gly Pro Gly Arg Ala Phe Arg Thr Arg Glu Ile
   1 5 10 15
<210> 23
   <211> 15
   <212> PRT
   <213> Human immunodeficiency virus
<400> 23
   Gly Ile Ala Ile Gly Pro Gly Arg Thr Leu Tyr Ala Arg Glu Lys
   1               5                   10                  15
<210> 24
   <211> 15
   <212> PRT
   <213> Human immunodeficiency virus
<400> 24
   Ser Ile Thr Lys Gly Pro Gly Arg Val Ile Tyr Ala Thr Gly Gln
   1               5                   10                  15
<210> 25
   <211> 15
   <212> PRT
   <213> Human immunodeficiency virus
<400> 25
   Arg Val Thr Leu Gly Pro Gly Arg Val Trp Tyr Thr Thr Gly Glu
   1               5                   10                  15
<210> 26
   <211> 15
   <212> PRT -
   <213> Human immunodeficiency virus
<400> 26
   Ser Ile His Ile Gly Pro Gly Arg Ala Phe Tyr Ala Thr Gly Asp
   1               5                   10                  15
<210> 27
   <211> 15
   <212> PRT
   <213> Human immunodeficiency virus
<400> 27
   Ser Ile Tyr Ile Gly Pro Gly Arg Ala Phe His Thr Thr Gly Arg
   1 5 10 15
<210> 28
   <211> 15
   <212> PRT
   <213> Human immunodeficiency virus
<400> 28
   Arg Ile Gln Arg Gly Pro Gly Arg Ala Phe Val Thr Ile Gly Lys
   1 5 10 15
<210> 29
   <211> 15
   <212> PRT
   <213> Human immunodeficiency virus
<400> 29
   Gly Ile His Phe Gly Pro Gly Gln Ala Leu Tyr Thr Thr Gly Ile
   1 5 10 15
<210> 30
   <211> 15
   <212> PRT
   <213> Human immunodeficiency virus
<400> 30
   Ser Thr Pro Ile Gly Leu Gly Gln Ala Leu Tyr Thr Thr Arg Gly
   1 5 10 15
<210> 31
   <211> 15
   <212> PRT
   <213> Human immunodeficiency virus
<400> 31
   Ser Thr Pro Ile Gly Leu Gly Gln Ala Leu Tyr Thr Thr Arg Ile
   1 5 10 15
<210> 32
   <211> 15
   <212> PRT
   <213> Human immunodeficiency virus
<400> 32
   Arg Thr Pro Thr Gly Leu Gly Gln Ser Leu Tyr Thr Thr Arg Ser
   1 5 10 15
<210> 33
   <211> 16
   <212> PRT
   <213> Hepatitis C virus
<400> 33
<210> 34
   <211> 16
   <212> PRT
   <213> Hepatitis C virus
<400> 34
<210> 35
   <211> 10
   <212> PRT
   <213> Hepatitis C virus
<400> 35
   Leu Met Gly Tyr Ile Pro Leu Val Gly Ala
   1 5 10
<210> 36
   <211> 17
   <212> PRT
   <213> Human
<220>
   <221> X
   <222> (7)..(9)
   <223> Xaa
<400> 36
<210> 37
   <211> 12
   <212> PRT
   <213> Chlamydia trachomatis
<400> 37
   Thr Ile Asn Lys Pro Lys Gly Tyr Thr Gly Lys Glu
   1 5 10
<210> 38
   <211> 9
   <212> PRT
   <213> Human immunodeficiency virus
<400> 38
   His Ile Val Trp Ala Ser Arg Phe Leu
   1 5
<210> 39
   <211> 9
   <212> PRT
   <213> Human immunodeficiency virus
<400> 39
   Ile Leu Gly Gln Leu Gln Pro Ser Leu
   1 5
<210> 40
   <211> 9
   <212> PRT
   <213> Human immunodeficiency virus
<400> 40
   Ser Leu Gln Thr Gly Ser Glu Glu Leu
   1 5
<210> 41
   <211> 9
   <212> PRT
   <213> Human immunodeficiency virus
<400> 41
   Glu Leu Arg Ser Leu Tyr Asn Thr Val
   1 5
<210> 42
   <211> 9
   <212> PRT
   <213> Human immunodeficiency virus
<400> 42
   Ser Leu Tyr Asn Thr Val Ala Thr Leu
   1 5
<210> 43
   <211> 9
   <212> PRT
   <213> Human immunodeficiency virus
<400> 43
   Glu Ile Lys Asp Thr Lys Glu Ala Leu
   1 5
<210> 44
   <211> 9
   <212> PRT
   <213> Human immunodeficiency virus
<400> 44
   Thr Leu Asn Ala Trp Val Lys Val Val
   1 5
<210> 45
   <211> 9
   <212> PRT
   <213> Human immunodeficiency virus
<400> 45
   Asp Leu Asn Thr Met Leu Asn Thr Val
   1               5
<210> 46
   <211> 9
   <212> PRT
   <213> Human immunodeficiency virus
<400> 46
   Asp Ile Ala Gly Thr Thr Ser Thr Leu
   1               5
<210> 47
   <211> 9
   <212> PRT
   <213> Human immunodeficiency virus
<400> 47
   Thr Leu Gln Glu Gln Ile Gly Trp Met
   1               5
<210> 48
   <211> 9
   <212> PRT
   <213> Human immunodeficiency virus
<400> 48
   Glu Ile Tyr Lys Arg Trp Ile Leu Leu
   1               5
<210> 49
   <211> 9
   <212> PRT
   <213> Human immunodeficiency virus
<400> 49
   Ile Ile Leu Gly Leu Asn Lys Ile Val
   1               5
<210> 50
   <211> 8
   <212> PRT
   <213> Human immunodeficiency virus
<400> 50
   Phe Met Met Thr Ala Cys Gly Val
   1               5
<210> 51
   <211> 9
   <212> PRT
   <213> Human immunodeficiency virus
<400> 51
   Val Leu Ala Glu Ala Met Ser Gln Val
   1               5
<210> 52
   <211> 9
   <212> PRT
   <213> Human immunodeficiency virus
<400> 52
   Pro Ile Asp Lys Glu Leu Tyr Pro Leu
   1               5
<210> 53
   <211> 9
   <212> PRT
   <213> Human immunodeficiency virus
<400> 53
   Leu Leu Thr Gln Ile Gly Cys Thr Leu
   1               5
<210> 54
   <211> 9
   <212> PRT
   <213> Human immunodeficiency virus
<400> 54
   Pro Ile Glu Thr Val Pro Val Lys Leu
   1               5
<210> 55
   <211> 9
   <212> PRT
   <213> Human immunodeficiency virus
<400> 55
   Ala Leu Val Glu Ile Cys Thr Glu Met
   1               5
<210> 56
   <211> 9
   <212> PRT
   <213> Human immunodeficiency virus
<400> 56
   Gly Ile Arg Tyr Gln Tyr Asn Val Leu
   1               5
<210> 57
   <211> 9
   <212> PRT
   <213> Human immunodeficiency virus
<400> 57
   Tyr Ile Tyr Gln Tyr Met Asp Asp Leu
   1               5
<210> 58
   <211> 9
   <212> PRT
   <213> Human immunodeficiency virus
<400> 58
   Lys Ile Glu Glu Leu Arg Gln His Leu
   1               5
<210> 59
   <211> 9
   <212> PRT
   <213> Human immunodeficiency virus
<400> 59
   Glu Leu His Pro Asp Lys Trp Thr Val
   1 5
<210> 60
   <211> 9
   <212> PRT
   <213> Human immunodeficiency virus
<400> 60
   Asp Ile Gln Lys Leu Val Gly Lys Leu
   1 5
<210> 61
   <211> 9
   <212> PRT
   <213> Human immunodeficiency virus
<400> 61
   Lys Leu Lys Arg Gly Thr Lys Ala Leu
   1 5
<210> 62
   <211> 9
   <212> PRT
   <213> Human immunodeficiency virus
<400> 62
   Glu Leu Ala Glu Asn Arg Glu Ile Leu
   1 5
<210> 63
   <211> 9
   <212> PRT
   <213> Human immunodeficiency virus
<400> 63
   Ile Leu Lys Glu Pro Val His Gly Val
   1 5
<210> 64
   <211> 8
   <212> PRT
   <213> Human immunodeficiency virus
<400> 64
   Pro Leu Val Lys Leu Trp Tyr Leu
   1               5
<210> 65
   <211> 9
   <212> PRT
   <213> Human immunodeficiency virus
<400> 65
   Glu Leu Gln Ala Ile Tyr Leu Ala Leu
   1               5
<210> 66
   <211> 9
   <212> PRT
   <213> Human immunodeficiency virus
<400> 66
   Tyr Leu Ala Leu Gln Asp Ser Gly Leu
   1               5
<210> 67
   <211> 9
   <212> PRT
   <213> Human immunodeficiency virus
<400> 67
   Ala Leu Gln Asp Ser Gly Leu Glu Val
   1               5
<210> 68
   <211> 9
   <212> PRT
   <213> Human immunodeficiency virus
<400> 68
   Leu Ile Lys Lys Glu Lys Val Tyr Leu
   1               5
<210> 69
   <211> 9
   <212> PRT
   <213> Human immunodeficiency virus
<400> 69
   His Leu Glu Gly Lys Val Ile Leu Val
   1               5
<210> 70
   <211> 9
   <212> PRT
   <213> Human immunodeficiency virus
<400> 70
   Glu Leu Lys Lys Ile Ile Gly Gln Val
   1               5
<210> 71
   <211> 9
   <212> PRT
   <213> Human immunodeficiency virus
<400> 71
   Pro Leu Trp Lys Gly Pro Ala Lys Leu
   1               5
<210> 72
   <211> 9
   <212> PRT
   <213> Human immunodeficiency virus
<400> 72
   Leu Leu Trp Lys Gly Glu Gly Ala Val
   1               5
<210> 73
   <211> 9
   <212> PRT
   <213> Human immunodeficiency virus
<400> 73
   Ile Ile Arg Asp Tyr Gly Lys Gln Met
   1               5
<210> 74
   <211> 9
   <212> PRT
   <213> Human immunodeficiency virus
<400> 74
   Met Ile Val Trp Gln Val Asp Arg Met
   1 5
<210> 75
   <211> 9
   <212> PRT
   <213> Human immunodeficiency virus
<400> 75
   Arg Ile Arg Thr Trp Lys Ser Leu Val
   1 5
<210> 76
   <211> 9
   <212> PRT
   <213> Human immunodeficiency virus
<400> 76
   Asp Leu Ala Asp Gln Leu Ile His Leu
   1 5
<210> 77
   <211> 9
   <212> PRT
   <213> Human immunodeficiency virus
<400> 77
   Lys Ile Lys Pro Pro Leu Pro Ser Val
   1 5
<210> 78
   <211> 9
   <212> PRT
   <213> Human immunodeficiency virus
<400> 78
   Met Ile Cys Ser Ala Thr Glu Lys Leu
   1 5
<210> 79
   <211> 9
   <212> PRT
   <213> Human immunodeficiency virus
<400> 79
   Ile Ile Leu Ser Trp Asp Gln Ser Leu
   1 5
<210> 80
   <211> 9
   <212> PRT
   <213> Human immunodeficiency virus
<400> 80
   Lys Leu Thr Ser Leu Cys Val Ser Leu
   1 5
<210> 81
   <211> 9
   <212> PRT
   <213> Human immunodeficiency virus
<400> 81
   Lys Leu Thr Ser Cys Asn Thr Ser Val
   1 5
<210> 82
   <211> 9
   <212> PRT
   <213> Human immunodeficiency virus
<400> 82
   Val Ile Thr Gln Ala Cys Pro Lys Val
   1 5
<210> 83
   <211> 9
   <212> PRT
   <213> Human immunodeficiency virus
<400> 83
   Ile Ile Val Gln Leu Asn Thr Ser Val
   1 5
<210> 84
   <211> 9
   <212> PRT
   <213> Human immunodeficiency virus
<400> 84
   Thr Leu Lys Gln Ile Ala Ser Lys Leu
   1 5
<210> 85
   <211> 9
   <212> PRT
   <213> Human immunodeficiency virus
<400> 85
   Ala Ile Ile Asn Met Trp Gln Lys Val
   1 5
<210> 86
   <211> 9
   <212> PRT
   <213> Human immunodeficiency virus
<400> 86
   Phe Leu Gly Ala Ala Gly Ser Thr Met
   1 5
<210> 87
   <211> 9
   <212> PRT
   <213> Human immunodeficiency virus
<400> 87
   Thr Leu Thr Val Gln Ala Arg Gln Leu
   1 5
<210> 88
   <211> 9
   <212> PRT
   <213> Human immunodeficiency virus
<400> 88
   Gly Ile Val Gln Gln Gln Asn Asn Leu
   1 5
<210> 89
   <211> 9
   <212> PRT
   <213> Human immunodeficiency virus
<400> 89
   Ala Ile Glu Ala Gln Gln His Leu Leu
   1 5
<210> 90
   <211> 9
   <212> PRT
   <213> Human immunodeficiency virus
<400> 90
   Gln Leu Gln Ala Arg Ile Leu Ala Val
   1 5
<210> 91
   <211> 9
   <212> PRT
   <213> Human immunodeficiency virus
<400> 91
   Arg Ile Leu Ala Val Glu Arg Tyr Leu
   1 5
<210> 92
   <211> 9
   <212> PRT
   <213> Human immunodeficiency virus
<400> 92
   Gly Ile Trp Gly Cys Ser Gly Lys Leu
   1 5
<210> 93
   <211> 9
   <212> PRT
   <213> Human immunodeficiency virus
<400> 93
   Gln Ile Trp Asn His Thr Thr Trp Met
   1 5
<210> 94
   <211> 9
   <212> PRT
   <213> Human immunodeficiency virus
<400> 94
   Tyr Ile Lys Leu Phe Ile Met Ile Val
   1 5
<210> 95
   <211> 9
   <212> PRT
   <213> Human immunodeficiency virus
<400> 95
   Phe Ile Met Ile Val Gly Gly Leu Val
   1 5
<210> 96
   <211> 9
   <212> PRT
   <213> Human immunodeficiency virus
<400> 96
   Met Ile Val Gly Gly Leu Val Gly Leu
   1 5
<210> 97
   <211> 9
   <212> PRT
   <213> Human immunodeficiency virus
<400> 97
   Gly Leu Arg Ile Val Phe Ala Val Leu
   1 5
<210> 98
   <211> 9
   <212> PRT
   <213> Human immunodeficiency virus
<400> 98
   Ser Ile Arg Leu Val Asn Gly Ser Leu
   1 5
<210> 99
   <211> 9
   <212> PRT
   <213> Human immunodeficiency virus
<400> 99
   Arg Leu Val Asn Gly Ser Leu Ala Leu
   1 5
<210> 100
   <211> 9
   <212> PRT
   <213> Human immunodeficiency virus
<400> 100
   Ser Leu Ala Leu Ile Trp Asp Asp Leu
   1 5
<210> 101
   <211> 9
   <212> PRT
   <213> Human immunodeficiency virus
<400> 101
   Leu Ile Trp Asp Asp Leu Arg Ser Leu
   1 5
<210> 102
   <211> 9
   <212> PRT
   <213> Human immunodeficiency virus
<400> 102
   Arg Leu Arg Asp Leu Leu Leu Ile Val
   1 5
<210> 103
   <211> 9
   <212> PRT
   <213> Human immunodeficiency virus
<400> 103
   Leu Leu Leu Ile Val Thr Arg Ile Val
   1               5
<210> 104
   <211> 9
   <212> PRT
   <213> Human immunodeficiency virus
<400> 104
   Leu Ile Val Thr Arg Ile Val Glu Leu
   1               5
<210> 105
   <211> 9
   <212> PRT
   <213> Human immunodeficiency virus
<400> 105
   Leu Leu Gln Tyr Trp Ser Gln Glu Leu
   1               5
<210> 106
   <211> 9
   <212> PRT
   <213> Human immunodeficiency virus
<400> 106
   Glu Leu Lys Asn Ser Ala Val Ser Leu
   1               5
<210> 107
   <211> 9
   <212> PRT
   <213> Human immunodeficiency virus
<400> 107
   Leu Leu Asn Ala Thr Ala Ile Ala Val
   1               5
<210> 108
   <211> 9
   <212> PRT
   <213> Human immunodeficiency virus
<400> 108
   Ala Ile Ile Arg Ile Leu Gln Gln Leu
   1 5
<210> 109
   <211> 9
   <212> PRT
   <213> Human immunodeficiency virus
<400> 109
   Ile Ile Arg Ile Leu Gln Gln Leu Leu
   1 5
<210> 110
   <211> 9
   <212> PRT
   <213> Human immunodeficiency virus
<400> 110
   Tyr Leu Gly Arg Ser Ala Glu Pro Val
   1 5
<210> 111
   <211> 9
   <212> PRT
   <213> Human immunodeficiency virus
<400> 111
   Gln Ile Leu Val Glu Ser Pro Thr Val
   1 5
<210> 112
   <211> 9
   <212> PRT
   <213> Human immunodeficiency virus
<400> 112
   Ile Leu Val Glu Ser Pro Thr Val Leu
   1 5
<210> 113
   <211> 9
   <212> PRT
   <213> Human immunodeficiency virus
<400> 113
   Gln Ile Ala Ile Val Ala Ala Leu Val
   1 5
<210> 114
   <211> 9
   <212> PRT
   <213> Human immunodeficiency virus
<400> 114
   Gly Met Asp Asp Pro Glu Lys Glu Val
   1 5